# EUROPÄISCHE PATENTSCHRIFT

(11) **EP 3 740 143 B1**
(45) Veröffentlichungstag und Bekanntmachung des Hinweises auf die Patenterteilung: **07.06.2023**
(21) Anmeldenummer: 19701483.0
(22) Anmeldetag: 18.01.2019
(51) Int. Cl.: A61B 17/22

(54) **VORRICHTUNG ZUR ZERTRÜMMERUNG EINES KÖRPERSTEINS**
DEVICE FOR THE FRAGMENTATION OF A CALCULUS
DISPOSITIF POUR LA DÉSINTÉGRATION D'UNE PIERRE CORPORELLE

(30) Priorität: 19.01.2018 DE 102018101215
(43) Veröffentlichungstag der Anmeldung: 25.11.2020
(73) Patentinhaber: Ferton Holding S.A., 2800 Delémont (CH)
(72) Erfinder: BIONDA, Pierre-Alain, 74930 Arbusigny (FR); GIROD, Jean-Yves, 1196 Gland (CH); EVANS, Gary, 74200 Allinges (FR)
(74) Vertreter: Müller Schupfner & Partner Patent- und Rechtsanwaltspartnerschaft mbB
(86) Internationale Anmeldenummer: PCT/EP2019/051283
(87) Internationale Veröffentlichungsnummer: WO 2019/141822

(56) Entgegenhaltungen:
- CN-B- 104 473 674
- DE-C1- 10 029 580
- US-A1- 2004 010 267
- US-A1- 2005 209 620
- US-A1- 2014 336 665

## Beschreibung

Die vorliegende Erfindung betrifft eine Vorrichtung zur Zertrümmerung eines Körpersteins.

Für die Entfernung von Körpersteinen, beispielsweise aus Körperhöhlen, ist es erforderlich, diese zunächst zu zerkleinern, wobei die Zerkleinerung in kleine, spontan abgangsfähige oder direkt aus dem Körper ausspülbare Partikel vorgenommen wird. Die Zerkleinerung der Körpersteine wird dabei z. B. durch mechanische Druck- und Zugspannungen vorgenommen, die bei der intrakorporalen Lithotripsie mit einem proximalen Ende einer als Wellenleiter dienenden (Metall-)Sonde auf die Körpersteine ausgeübt werden. Solche Spannungen führen zu einem Absprengen von Fragmenten aus der Oberfläche des Körpersteins und bewirken schließlich dessen Zertrümmerung.

Bekannt sind zum Beispiel intrakorporale Lithotripter, bei welchen die Sonde durch einen elektrisch angesteuerten Ultraschallwandler zu Longitudinalschwingungen angeregt wird. Mit einer Vorrichtung dieser Art lassen sich die Körpersteine in aller Regel zu sehr feinen Fragmenten zertrümmern.

Bekannt sind weiter Lithotripter, bei welchen das distale Ende der Sonde mit einem pneumatisch angetriebenen Schlagteil beaufschlagt wird. Solche Stoßwellenlithotripter, die bei anderen Ausführungen auch einen elektrischen Antrieb des Schlagteils aufweisen können, erlauben höhere maximale Amplituden bzw. Impulse der Sondenspitze, sind allerdings nicht optimal geeignet für eine sehr feine Zertrümmerung. Aus der EP 1 163 884 B1 ist beispielsweise eine Vorrichtung bekannt, bei welcher durch eine spezielle elektrische Ansteuerung des Ultraschallwandlers zwischen einer periodischen Schwingungsanregung der Sonde und einer impulsförmigen Schwingungsanregung der Sonde aufgrund eines Spannungsimpulses umgeschaltet werden kann. Ferner ist aus der DE 100 29 580 C1 bekannt, zwischen einem ersten Betriebszustand, in dem eine periodische Auslenkung der Sonde veranlasst wird, und einem zweiten Betriebszustand, in dem eine impulsförmige Auslenkung der Sonde veranlasst wird, zu wechseln.

Die bekannten Vorrichtungen haben allerdings den Nachteil, dass es nicht mit ein und derselben Vorrichtung möglich ist, sowohl große und harte Körpersteine zu zertrümmern, was eine hohe maximale Amplitude der bzw. einen großen Impuls über die Sonde erfordert, als auch gleichzeitig eine sehr feine Körpersteinzertrümmerung zu ermöglichen, was geringe Amplituden bzw. Auslenkungen der Sonde bei gleichzeitig sehr hohen Frequenzen erfordert.

Aus der US 2014 033 6665 A1 ist ein Lithotripter bekannt, bei der eine erste Antriebseinrichtung und eine zweite Antriebseinrichtung ein Schlagelement zur Bewegung antreiben und über ein Bauteil miteinander verbunden und somit gekoppelt sind.

Die CN 104 473 674 B beschreibt einen weiteren Lithotripter, der ein Schlagteil aufweist, das ballistisch angetrieben wird.

Es ist daher Aufgabe der vorliegenden Erfindung, eine Vorrichtung der eingangs genannten Art derart auszubilden, dass damit unter Berücksichtigung der Vor- und Nachteile dieser bekannten Verfahren eine flexiblere und effizientere Steinzertrümmerung durchführbar ist.

Diese Aufgabe wird durch eine Vorrichtung zur Zertrümmerung eines Körpersteins gemäß Anspruch 1 gelöst. Weitere Vorteile und Merkmale ergeben sich aus den Unteransprüchen sowie der Beschreibung und den beigefügten Figuren.

Erfindungsgemäß ist eine Vorrichtung zur Zertrümmerung eines Körpersteins, insbesondere ein Lithotripter, mit einer Sonde und einer Antriebseinheit zur Auslenkung der Sonde bzw. zur Einleitung eines Stoßimpulses in die Sonde entlang deren Längserstreckung, vorgesehen, wobei die Antriebseinheit eine erste Antriebseinrichtung zur periodischen Auslenkung der Sonde und eine zweite Antriebseinrichtung zur impulsförmigen Auslenkung der Sonde umfasst, wobei die Antriebseinheit derart gestaltet ist, dass eine von der zweiten Antriebseinrichtung ausgehende Wirkung auf die erste Antriebseinrichtung reduziert wird. Unter "Auslenkung" versteht die Erfindung auch die Einleitung bzw. Übertragung einer Stoßwelle bzw. eines Impulses in die bzw. über die Sonde auf den Körperstein.

Im Gegensatz zum Stand der Technik lässt sich mit der erfindungsgemäßen Reduktion der von der zweiten Antriebseinrichtung ausgehenden Wirkung auf die erste Antriebseinheit diese im Betrieb vor Schädigungen schützen. Andernfalls wäre mit solchen Schädigungen, zumindest auf Dauer, wegen der intensiven Antriebswirkung der zweiten Antriebseinrichtung auf die Sonde, die auch im Bereich der ersten Antriebseinrichtung bemerkbar bzw. spürbar ist, zu rechnen. Infolgedessen ist es beispielsweise nicht länger zwingend erforderlich, zum Schutz der ersten Antriebseinrichtung, die zweite Antriebseinrichtung mechanisch von der Antriebseinheit und/oder den Kontakt zur Sonde zu lösen, wenn nur die zweite Antriebseinrichtung betrieben werden soll. Mit anderen Worten: Die erste Antriebseinrichtung muss nicht von der zweiten Antriebseinrichtung bzw. der Sonde gelöst werden, wenn z. B. zwischen einem ersten Betriebsmodus, in dem nur die periodische Auslenkung veranlasst wird, und einem zweiten Betriebsmodus, in dem nur die impulsförmige Auslenkung veranlasst wird, gewechselt bzw. umgeschaltet wird. Darüber hinaus gestattet es die erfindungsgemäße Gestaltung sogar, periodische und impulsförmige Auslenkungen gleichzeitig zu realisieren.

Grundsätzlich ist unter einer Gestaltung zur Reduktion der von der zweiten Antriebseinrichtung ausgehenden Wirkung jede Maßnahme an der Antriebseinheit zu verstehen, mit der sich die Wirkung auf die erste Antriebseinrichtung reduzieren lässt. D. h. es ist beispielsweise eine Dämpfung einer von der zweiten Antriebseinrichtung ausgehenden impulsförmigen Schwingung und der damit verbundenen Kräften vor ihrem Eintritt in die erste Antriebseinrichtung gemeint. Diese Reduktion lässt sich beispielsweise durch Vergleich mit einer Antriebseinheit, bei der die Maßnahme nicht und/oder eine gegenteilige Maßnahme vorgenommen wurde, feststellen. Denkbar ist dabei beispielsweise, dass mit der Maßnahme eine von der zweiten Antriebseinrichtung angeregte impulsförmige Schwingung bzw. Kompressionswelle, insbesondere in einem kraftübertragenden Schwingteil, um mehr als 50 %, bevorzugt mehr als 70 % und besonders bevorzugt mehr als 85 % (bezogen auf Amplitude beim Einleiten in das Schwingteil) gedämpft wird, bevor sie in die erste Antriebseinrichtung eingekoppelt bzw. eingeleitet wird. Bevorzugt dient die Maßnahme einer Dämpfung von impulsförmigen Schwingungen.

Gemäß einer bevorzugten Ausführungsform ist es vorgesehen, dass die Antriebseinheit derart gestaltet ist, dass die periodische Auslenkung und die impulsförmige Auslenkung überlagerbar sind. Im Gegensatz zu den aus dem Stand der Technik bekannten Vorrichtungen zur Zertrümmerung von Körpersteinen ist es somit möglich, die Antriebseinheit zur Überlagerung von periodischen und impulsförmigen Auslenkungen zu nutzen. Mit anderen Worten: Periodische und impulsförmige Auslenkungen der Sonde können, zumindest zeitweise, gleichzeitig veranlasst werden. Dabei wirken die erste und die zweite Antriebseinrichtung gleichzeitig auf die Sonde ein, d. h. die erste und die zweite Antriebseinrichtung sind gleichzeitig im Betrieb.

Beispielsweise ist es hierbei nicht erforderlich, die erste Antriebseinrichtung und die zweite Antriebseinrichtung abwechselnd an die Sonde anbinden zu müssen. Es hat sich dabei gezeigt, dass sich mit einer Auslenkung, die sowohl einen periodischen als auch einen impulsförmigen Anteil hat, die Effizienz der Zertrümmerung signifikant erhöhen lässt und zwar in einem Maß, das erheblich über die sukzessive Anwendung beider Betriebsmodi hinausgeht. Dabei ist die Vorrichtung nach einer Ausführungsform dazu ausgelegt, zwischen einem ersten Betriebsmodus, in dem nur die periodische Auslenkung veranlasst wird, und einem zweiten Betriebsmodus, in dem nur die impulsförmige Auslenkung veranlasst wird, sowie einem dritten Betriebsmodus, in dem die periodische und die impulsförmige Auslegung überlagert sind, wahlweise bzw. bedarfsabhängig wechseln zu können.

Grundsätzlich ist unter einer periodischen Auslenkung eine Schwingung, insbesondere eine Ultraschallschwingung zu verstehen, die in der Sonde eine stehende Welle veranlasst, während unter einer impulsförmigen Auslenkung beispielswiese eine schlagende bzw. hämmernde Auslenkung zu verstehen ist. Vorzugsweise ist dabei eine Auslenkung, die von der zweiten Antriebseinrichtung veranlasst wird, um ein vielfaches größer als die Auslenkung die von der ersten Antriebseinrichtung veranlasst wird. Beispielsweise ist die Sonde nadelförmig und bevorzugt hohl ausgeformt, wobei die nadelförmige Form die Längserstreckung der Sonde vorgibt. D. h. eine Auslenkung entlang der Längserstreckung entspricht einer translatorischen Vor- und Rückbewegung der Sonde. Weiterhin ist es besonders bevorzugt vorgesehen, dass die Sonde austauschbar ist bzw. lösbar mit der Antriebseinheit verbindbar ist. Beispielsweise ist die Sonde über ein Gewinde an die Antriebseinheit anschraubbar.

Vorzugsweise sind die erste Antriebseinrichtung und die zweite Antriebseinrichtung entlang einer Längsachse, die im montierten Zustand der Sonde parallel zu deren Längserstreckung verläuft, versetzt zueinander angeordnet, wobei die zweite Antriebseinrichtung in der Antriebseinheit näher an der Sonde angeordnet ist als die erste Antriebseinrichtung. Dadurch muss mit Vorteil die die impulsförmige Auslenkung veranlassende Kraft auf ihrem Weg zur Sonde nicht an der ersten Antriebseinrichtung vorbeigeführt werden. Vorstellbar ist aber auch, dass die erste Antriebeinrichtung näher an der Sonde angeordnet ist als die zweite Antriebseinrichtung. Die erste und zweite Antriebeinrichtung wirken jeweils mittels parallel zur Längsachse verlaufender Kräfte auf die Sonde ein, um die gewünschte Überlagerung der periodischen und der impulsförmigen Auslenkung zu realisieren.

Erfindungsgemäß ist es vorgesehen, dass die erste Antriebseinrichtung, vorzugsweise bestehend aus Piezokeramikelemente, über ein Schwingteil auf die Sonde einwirkt und die zweite Antriebseinrichtung über ein Prellkörper auf die Sonde einwirkt. Denkbar ist auch, dass die zweite Antriebseinrichtung unmittelbar auf die Sonde einwirkt. Vorzugsweise wirken die erste Antriebseinrichtung und die zweite Antriebseinrichtung separat bzw. isoliert, d. h. nicht über ein gemeinsames Kraftflussmittel, auf die Sonde ein. Diese Entkopplung beim Einwirken der durch die erste Antriebseinrichtung und die zweite Antriebseinrichtung veranlassten Kräfte erweist sich als vorteilhaft, da dadurch ein unmittelbarer Einfluss der Kräfte, die z. B. die impulsförmigen Schwingungen bedingen, auf die erste Antriebseinrichtung reduziert werden können. Beispielsweise kann so die von der zweiten Antriebseinrichtung ausgehende impulsförmige Schwingung nur über das Gewinde zur ersten Antriebseinrichtung gelangen. Vorzugsweise erfolgt die Einleitung der Kraft, die von der zweiten Antriebseinrichtung ausgeht direkt auf die Sonde, beispielsweise auf ein Kragenelement der Sonde und nicht über das Gewinde, das die Sonde mit der Antriebseinheit verbindet. D. h. eine von der zweiten Antriebseinrichtung ausgehende Kraft wird direkt in die Sonde eingeleitet. Weiterhin ist es bevorzugt vorgesehen, dass das Schwingteil zur Ausbildung einer stehenden Welle elastisch in der Antriebseinheit gelagert ist. Dabei ist die Sonde mit dem Schwingteil vorzugsweise kraft- und/oder formschlüssig verbunden. Beispielsweise ist die Sonde mit dem Schwingteil verschraubt, wodurch sich bei einer Anregung durch die erste Antriebseinrichtung in Sonde und Schwingteil gleichermaßen eine stehende Welle ausbildet.

Erfindungsgemäß ist es vorgesehen, dass die erste Antriebseinrichtung über einen Übertragungsbereich eine die periodische Schwingung veranlassende Kraft auf das Schwingteil überträgt, wobei zur Dämpfung von impulsförmigen Schwingungen der Übertragungsbereich frequenzselektiv, insbesondere als Tiefpassfilter, ausgelegt ist. Dadurch ist es in vorteilhafter Weise möglich, dass impulsförmige Schwingungen, insbesondere diejenigen die von der zweiten Antriebseinrichtung beim gleichzeitigen Betrieb der ersten und der zweiten Antriebseinrichtung bedingt werden und ihren Weg über das Schwingteil zur ersten Antriebseinrichtung finden, gedämpft werden, bevor sie in die erste Antriebseinrichtung eindringen können. Es wird demnach verhindert, dass impulsförmige Schwingungen, insbesondere Druck- und Kraftspitzen, in die erste Antriebseinrichtung ungedämpft eingespeist werden. Infolgedessen lässt sich einer Schädigung der ersten Antriebseinrichtung entgegenwirken, mit der andernfalls bei einem dauerhaften Einleiten von impulsförmigen Schwingungen in die erste Antriebseinrichtung zu rechnen wäre.

Dabei ist unter einer frequenzselektiven Auslegung eine solche Gestaltung des Übertragungsbereichs, beispielsweise durch Form und/oder Material des Übertragungsbereichs, zu verstehen, durch die Schwingungen einer bestimmten Frequenz bzw. eines Frequenzbands mehr geschwächt werden als Schwingungen einer anderen Frequenz bzw. eines Frequenzbands. Die Ausgestaltung als Tiefpassfilter erweist sich insbesondere als vorteilhaft, weil die hochfrequenten Schwingungen, die aus den impulsförmigen Schwingungen der Schläge der zweiten Antriebseinheit entstehen, gedämpft oder sogar unterdrückt werden. Daher wird mit der frequenzselektiven Auslegung die Funktionsfähigkeit der Vorrichtung dauerhaft sichergestellt, insbesondere trotz der Belastungen, die durch die gleichzeitige Inbetriebnahme der ersten und der zweiten Antriebseinrichtung bedingt werden.

Erfindungsgemäß ist es vorgesehen, dass der Übertragungsbereich zwischen einem Übertragungskörper der ersten Antriebseinrichtung und dem Schwingteil eine Querschnittsverjüngung , beispielsweise in Form einer Unterlegscheibe, aufweist, wodurch die Wirkung, insbesondere Kraftwirkung, von der zweiten Antriebseinheit auf die erste Antriebseinheit reduziert wird. Mit der Querschnittsverjüngung lässt sich in vorteilhafter Weise sicherstellen, dass die Kraft bzw. Impulse für die periodische Auslenkung bzw. zur Bildung der stehenden Welle ungehindert auf das Schwingteil übertragen werden können, während ein großer Anteil der impulsförmigen Anregung an der Querschnittsverjüngung reflektiert wird und so nicht in die erste Antriebseinrichtung eingeleitet werden kann.

Unter einer Querschnittsverjüngung versteht der Fachmann insbesondere eine in Richtung der Längsachse abnehmende Querschnittsfläche, die zur Übertragung der von der ersten Antriebsrichtung ausgehenden Kraft auf das Schwingteil dient.

Dabei kann die Querschnittsverjüngung stufenförmig erfolgen und es ist auch vorstellbar, dass die Querschnittsverjüngung mehrstufig erfolgt. Weiterhin ist es vorstellbar, dass der Übertragungsbereich im montierten Zustand stegartig, beispielswiese in Form eines ringförmigen Stegs, zwischen der ersten Antriebseinrichtung und dem Schwingteil ausbildet ist. Dabei kann der Steg in einer senkrecht zur Längsachse verlaufenden Ebene unterbrochen oder durchgängig ausgestaltet sein.

Besonderes bevorzugt ist es vorgesehen, wenn der Übertragungsbereich durch eine Unterlegscheibe realisiert ist, die beispielsweise zwischen der ersten Antriebseinrichtung und dem Schwingteil im montierten Zustand verklemmt ist oder an die erste Antriebseinrichtung kraftschlüssig oder stoffschlüssig angebunden ist. Beispielsweise ist der Steg in einer senkrecht zur Längsachse verlaufenden Richtung gesehen im Wesentlichen auf Höhe der halben Ausdehnung der ersten Antriebseinrichtung in derselben Richtung angeordnet. Ferner ist es vorstellbar, dass mehrere Stege im Übertragungsbereich, beispielsweise konzentrisch zueinander, angeordnet sind.

Weiterhin ist es vorzugsweise vorgesehen, dass die Querschnittsverjüngung einen Wert zwischen 0,02 und 0,5, bevorzugt zwischen 0,02 und 0,25 und besonders bevorzugt zwischen 0,02 und 0,1 annimmt. Dabei wird unter der Querschnittsverjüngung insbesondere die flächenmäßige Abnahme in Richtung der Längsachse verstanden. D. h. zur quantitativen Angabe der Querschnittsverjüngung bestimmt sich die Querschnittsverjüngung als Verhältnis des senkrecht zur Längsachse bemessenen ersten Querschnitts im Übertragungsbereich zu einem senkrecht zur Längsachse bemessenen zweiten Querschnitts der ersten Antriebseinrichtung, insbesondere an deren dem Übertragungsbereich zugewandten Stirnseite. Um eine ausreichende dämpfende Wirkung zu erzielen, hat sich insbesondere die Querschnittsverjüngungen zwischen 0,02 und 0,1 als vorteilhaft erwiesen. Dabei ist der Übertragungsbereich noch so breit gestaltet, dass der Übertragungsbereich auf Grund der Schwingungsbelastung auf Dauer nicht deformiert bzw. geschädigt wird.

Dabei ist es vorstellbar, dass die erste Antriebseinrichtung in Richtung der Längsachse gesehen an beiden Stirnseiten jeweils ein Übertragungsbereich, insbesondere jeweils mit einer Querschnittsverjüngung, aufweist. Insbesondere ist es vorgesehen, dass der zur frequenzselektiven Übertragung ausgelegte Übertragungsbereich an einer der zweiten Antriebseinrichtung zugewandten Seite, insbesondere Stirnseite, der ersten Antriebseinrichtung bzw. einer der Sonde zugewandten Stirnseite ausgebildet ist. Als besonders vorteilhaft hat es sich erwiesen, wenn der Übertragungsbereich ausschließlich auf einer der der Sonde bzw. der zweiten Antriebseinrichtung zugewandten Stirnseite der ersten Antriebseinrichtung angeordnet ist.

Vorzugsweise ist es vorgesehen, dass eine Zusatzmasse zwischen der ersten Antriebseinrichtung und der zweiten Antriebseinrichtung vorgesehen ist, wobei vorzugsweise das Schwingteil einen Grundkörper aufweist, wobei das Schwingteil, insbesondere angrenzenden an den zur frequenzselektiven Übertragung ausgelegte Übertragungsbereich, einen vom Grundkörper senkrecht zur Längsachse abstehenden Vorsprung aufweist. Das Einführen der Zusatzmasse zwischen der ersten Antriebseinrichtung und der zweiten Antriebseinrichtung hat sich als wirkungsvolle Maßnahme erwiesen, mit der sich die Wirkung der zweiten Antriebseinrichtung auf die erste Antriebseinrichtung weiter dämpfen bzw. reduzieren lässt.. Vorzugsweise ist die Zusatzmasse dabei als integraler Bestandteil des Schwingteils ausgestaltet. Dabei ist es vorstellbar, dass sich der senkrecht zur Längsachse bemessene Querschnitt des Schwingteils im Bereich des Vorsprungs mehr als das 1,2- fache, bevorzugt mehr als das 1,5 fache und besonders bevorzugt mehr als das 1,8 - fache des Querschnitts des Schwingteiles außerhalb des Vorsprungs annimmt. Insbesondere bildet der Vorsprung eine Art Mitnehmer, über den das Schwingteil zur periodischen Schwingung angeregt bzw. bewegt wird. Vorzugsweise nimmt ein Verhältnis zwischen einer in Richtung der Längsachse bemessene Länge des Vorsprungs zu einer in Richtung der Längsachse bemessenen Länge bzw. Gesamtlänge des Schwingteils einen Wert zwischen 0,1 und 0,5, bevorzugt zwischen 0,25 und 0,4 und besonders bevorzugt zwischen 0,28 und 0,38 annimmt. Durch diese vergleichsweise große Dimensionierung des Vorsprungs wird mit Vorteil eine größere Masse bereitgestellt, durch die die impulsförmige Schwingung hindurchlaufen muss, bevor sie in die erste Antriebseinrichtung einkoppeln kann.

In einer weiteren Ausführungsform ist es vorgesehen, dass ein Hohlbereich in der Sonde und ein Hohlbereich im Schwingteil einen Hohlkanal, insbesondere einen Hohlkanal ohne Querschnittsveränderung, zum Absaugen von Körpersteinfragmenten bilden. Vorzugsweise geht der Hohlbereich in der Sonde ohne Querschnittsveränderung in den Hohlbereich des Schwingteils über. Hierzu sind das Schwingteil und die Sonde derart gestaltet, dass im angebundenen, insbesondere angeschraubten, Zustand der Hohlbereich in der Sonde fluchtend zum Hohlbereich des Schwingteils angeordnet ist. Über den Hohlkanal lassen sich dann im Betrieb mit Vorteil zertrümmerte Körpersteinfragmente absaugen.

Zweckmäßig ist es vorgesehen, dass die erste Antriebseinrichtung mindestens ein Piezoelement und/oder die zweite Antriebseinrichtung mindestens einen Elektromagneten, insbesondere in Gestalt einer elektrischen Spule, umfasst. Insbesondere bilden mehrere nebeneinander bzw. gestapelte Piezoelemente einen Piezostack, mit dem sich in vorteilhafter Weise zuverlässig Ultraschallschwingungen bereitstellen lassen. Allerdings sind die Piezoelelmente bruchanfällig, insbesondere gegen impulsförmige Schwingungen, die in den Piezostack eingeleitet werden. Insofern erweist sich die frequenzselektive Auslegung des Übertragungsbereichs für die Piezoelemente als besonders vorteilhaft, da so die vorzugsweise aus einer Keramik gefertigten Piezoelemente vor Brüchen oder anderen Schädigungen geschützt werden können. Mittels der zweiten Antriebseinrichtung lassen sich Projektile beschleunigen, wobei die Projektile vorzugsweise ein Gewicht zwischen 10 und 100 g aufweisen, und dabei lässt sich das Projektil mit einer Frequenz zwischen 1 und 30 Hz auf den Prellkörper beschleunigen. Besonders geeignete Frequenzen liegen zwischen 5 und 15 Hz. Dabei werden Aufschlaggeschwindigkeiten zwischen 1 und 5 m/s erzielt. Als Prellkörper ist vorzugsweise ein Amboss vorgesehen, der an der Sonde anliegt und den Stossimpuls auf die Sonde weiterleitet.

Vorzugsweise ist es vorgesehen, dass zwischen dem Übertragungsbereich und dem Piezoelement ein Übertragungskörper, vorzugsweise ein metallischer Übertragungskörper vorgesehen ist. Der Übertagungskörper bildet insbesondere in Richtung der Längsachse einen Abschluss der ersten Antriebseinrichtung. Der Übertragungskörper stellt sicher, dass der verjüngende Querschnitt des Übertragungsbereichs nicht unmittelbar an ein Piezoelement angrenzt. Schließlich ist eine großflächige und plane Kontaktfläche für das Piezoelement erforderlich, um ein ungewolltes Brechen des Piezoelements zu vermeiden. Diese Kontaktfläche kann mit dem Übertragungskörper bereitgestellt werden, so dass der verjüngende Querschnitt des Übertragungsbereichs keine Gefahr für die Piezoelemente darstellt.

Ein weiterer Aspekt der vorliegenden Offenbarung ist ein Verfahren zur Zertrümmerung eine Körpersteins mittels einer Sonde und einer Antriebseinheit zur Auslenkung der Sonde entlang deren Längsachs, insbesondere mittels einer Vorrichtung gemäß einem der vorhergehenden Ansprüche, wobei die Antriebseinheit einer erste Antriebseinrichtung und eine zweite Antriebseinrichtung umfasst, wobei die Sonde gleichzeitig von der ersten Antriebseinrichtung und der zweiten Antriebseinrichtung ausgelenkt wird. Alle für die erfindungsgemäße Vorrichtung beschriebenen Merkmale und deren Vorteile lassen sich sinngemäß ebenfalls auf das nicht beanspruchte Verfahren übertragen und andersherum.

Zweckmäßigerweise umfasst die Vorrichtung ein Handinstrument, welches ein Gehäuse aufweist, innerhalb dessen die Antriebseinheit angeordnet ist. Zweckmäßigerweise umfasst die Vorrichtung ebenfalls eine Versorgungseinheit, beispielsweise in Form einer stationären Einheit, welche über Versorgungsleitungen beispielsweise für Strom, Luft und/oder andere Medien mit dem Handinstrument verbunden ist. Mit Vorteil kann die stationäre Einheit auch ausgelegt sein, Betriebsmodi des Handinstruments oder dergleichen zu ändern.

Es versteht sich, dass auch am Handinstrument selbst Betriebsmodi und dergleichen verstellbar sein können. Mit Vorteil ist die Sonde mit einem Ende innerhalb eines Führungsrohrs bzw. Führungskanals aufgenommen, über welches eine Spitze der Sonde, also deren distales Ende, für eine Berührungsmöglichkeit mit einem zu zertrümmernden Stein nach vorne vorsteht. Zweckmäßigerweise ist das Führungsrohr so bemessen, dass zwischen ihm und der Sonde ein Ringkanal ausgebildet ist, an dem eine Saugpumpe anschließbar ist. Der Ringkanal bildet damit einen Ansaugkanal aus, welcher bei angeschlossener Saugpumpe zu Beginn der Steinzertrümmerung den Stein an dem Ende des Führungsrohrs festhält, so dass die an der Sondenspitze zur Übertragung kommende Stoßenergie eine genau gezielte Einwirkung auf den Stein erfahren kann. Daneben kann der Ringkanal einen Absaugkanal für Steinfragmente einer Größe kleiner als der Querschnitt des Ringkanals darstellen. Alternativ bevorzugt kann auch eine hohle Sonde innerhalb des Führungsrohrs angeordnet sein, wodurch dann durch die Sonde selbst ein Absaugkanal zur Verfügung gestellt ist. Entscheidend ist, dass die Antriebseinheit für eine periodische und gleichzeitig für eine impulsförmige Auslenkung der Sonde ausgelegt ist.

Insbesondere von Vorteil ist dabei, dass die impulsförmige Auslenkung größer ist als die periodische Auslenkung. Dabei ist mit Auslenkung der Weg bzw. die maximale Amplitude gemeint, um welche sich die Sonde bzw. die Sondenspitze hin- und herbewegt. Damit können die Vorteile einer Vorrichtung mit einem pneumatisch angetriebenen Schwingteil und die Vorteile einer Vorrichtung mit einem elektrisch angesteuerten Ultraschallwandler durch die erfindungsgemäße Antriebseinheit in einer Vorrichtung kombiniert werden.

Vorteilhafterweise ist der Piezostack im Wesentlichen rohrförmig, weist also mittig eine Öffnung oder einen Durchgang entlang seiner Längsachse auf. Zweckmäßigerweise ist innerhalb der Öffnung des Piezostacks das bereits erwähnte Führungsrohrangeordnet bzw. es wird durch die Öffnung der Führungskanal festgelegt, innerhalb dessen wiederum das Schwingteil angeordnet ist.

Die Längsrichtung des Piezostacks erstreckt sich also mit Vorteil entlang der Längsachse der Vorrichtung. Zweckmäßigerweise ist der Querschnitt bzw. die Außenkontur des Piezostacks quer zur Längsrichtung bzw. zur Axialrichtung im Wesentlichen rund, insbesondere im Wesentlichen kreisrund. Denkbar sind auch beliebige andere Formen, beispielsweise ovale oder eckige Ausführungen. Es versteht sich, dass die erwähnten geometrischen Merkmale in gleicher Weise für jedes der Piezoelemente gelten. Dabei handelt es sich bei einem Piezoelement grundsätzlich um ein Bauteil, das den Piezoeffekt nutzt, um durch Anlegen einer elektrischen Spannung, beispielsweise über zwei Elektroden, eine mechanische Bewegung auszuführen. Die relative Ausdehnung bzw. mechanische Bewegung des Piezoelements ist proportional zur elektrischen Feldstärke, welche umso höher ist, je kleiner der Abstand der zwei Elektroden bei gegebener Spannung ist.

Bei mehrlagigen Piezoelementen, also bei Piezostacks, wird dies bevorzugt durch eine stapelweise Anordnung mit dazwischen liegenden Elektroden erreicht. Eine typische Anzahl von Piezoelementen liegt zwischen zwei und acht Scheiben, wobei insbesondere ausschließlich eine gerade Anzahl an Scheiben verwendet wird. Vorteilhafterweise sind die Plus- und Minuselektroden außen am Piezostack verschaltet bzw. elektrisch verbunden. Mit Vorteil weisen der Piezostack bzw. die Piezoelemente in der runden bzw. in der im Wesentlichen kreisrunden Ausführungsform einen Außendurchmesser von etwa 15 bis 30 mm, ganz bevorzugt von etwa 20 auf. Ein Innendurchmesser des Piezostacks bzw. der Piezoelemente weist bevorzugt einen Innendurchmesser von 5 bis 10 mm, ganz bevorzugt einen Durchmesser von etwa 8 bis 9 mm auf. Eine Dicke der Scheibe beträgt bevorzugt etwa 3 bis 7 mm, ganz bevorzugt etwa 4 bis 6 mm. Eine besonders bevorzugte Ausführungsform des Piezostacks besteht aus vier (4) Piezoelementen, weist einen Außendurchmesser von etwa 20 mm und einen Innendurchmesser von etwa 8,5 mm auf, wobei die Dicke eines Piezoelements mit Vorteil in etwa 4 mm beträgt.

Zweckmäßigerweise ist die Vorrichtung derart gestaltet, dass der Piezostack entlang der Längsachse eine Länge von etwa 10 bis 50 mm aufweist. Bevorzugt beträgt die Länge etwa 15 bis 30 mm, besonders bevorzugt etwa 15 bis 18 mm. Versuche haben gezeigt, dass sich mit einem Piezostack dieser Länge (in Kombination mit den bereits vorher genannten Innen- und Außendurchmessern) eine optimale periodische Auslenkung der Sonde erzielen lässt. Mit Vorteil ist der Piezostack mit einer Isolationsschicht, welche gleichzeitig als ein Gehäuse bzw. ein Gehäuseelement dienen kann, umschlossen. Es versteht sich, dass die Piezoelemente nicht alle die gleiche Dicke aufweisen müssen, sondern dass die Dicken der Piezoelemente bevorzugt auch unterschiedlich ausgeführt sein können. Ebenfalls bevorzugt können Piezoelemente unterschiedlicher Materialien verwendet werden. Dies gilt in gleicher Weise für den Außendurchmesser und/oder für den Innendurchmesser, welcher bzw. welche entlang der Axialrichtung ebenfalls nicht konstant sein muss bzw. müssen.

Bevorzugterweise ist die Vorrichtung derart gestaltet, dass bei der periodischen Auslenkung das distale Ende der Sonde im Wesentlichen eine sinusförmige Bewegung in Richtung der Längsachse ausführt wird, welche für die Zertrümmerung des Körpersteins ausgelegt ist und dass eine Frequenz der periodischen Auslenkung bei etwa 15 bis 50 kHz, besonders bevorzugt bei 25 kHz, liegt und dass die Auslenkung des distalen Endes (peak to peak) der Sonde in der Längserstreckung in einem Bereich von etwa 5 bis 100 µm liegt.

Weiter ist die Vorrichtung vorzugsweise derart gestaltet, dass bei der impulsförmigen Auslenkung am distalen Ende ein Druckimpuls eingeleitet wird, welcher für die Zertrümmerung des Körpersteins ausgelegt ist und dass eine Frequenz der impulsförmigen Auslenkung bei etwa 1 bis 100 Hz, vorzugsweise zwischen 1 bis 30 Hz, liegt und dass die Auslenkung des distalen Endes der Sonde aus der Ruhelage in der Axialrichtung in einem Bereich von etwa 50 bis 200 µm, vorzugsweise zwischen 75 µm und 150 µm, liegt. Im Kombinationsbetrieb, wenn also beide Antriebe gleichzeitig aktiviert sind, werden bevorzugt Amplituden aus der Ruhelage von 75 bis 250 µm erreicht, idealerweise Amplituden zwischen 100 und 150µm. Zweckmäßigerweise findet also die periodische Anregung mit einer um Größenordnungen höheren Frequenz statt als die impulsförmige Auslenkung. Vorteilhafterweise liegt die periodische Auslenkung bzw. deren Frequenz im Bereich bekannter Ultraschallwandler. Zweckmäßigerweise ist die Auslenkung bzw. die maximale Amplitude der Sonde bei der impulsförmigen Auslenkung deutlich höher als bei der periodischen Auslenkung. Mit Vorteil liegt die Auslenkung bei der impulsförmigen Auslenkung im Bereich pneumatisch angetriebener Schwingteile. Durch die an dem Piezostack angebrachten Elektroden kann eine Spannung angelegt werden, wodurch ein elektrisches Feld entsteht.

Die Ausdehnung des Piezostacks bzw. der Piezoelemente ist unter anderem abhängig von der elektrischen Feldstärke. Durch die Höhe der angelegten Spannung bzw. die Höhe des elektrischen Feldes können die Auslenkung, also die maximale Amplitude. Grundsätzlich sind für die Ansteuerung des Piezostacks bzw. der Piezoelemente aus dem Stand der Technik bekannte Schaltungsanordnungen verwendbar. Diese können in das Handinstrument integriert sein. Besonders bevorzugt sind sie aber Teil der bereits angesprochenen stationären Einheit.

Aufgrund der hohen Wärmentwicklung der ersten und zweiten Antriebseinrichtung ist es vorzugsweise vorgesehen, das Handinstrument und/oder die Arbeitseinheit zu kühlen, insbesondere mit einer Flüssigkeitskühlung. Zu diesem Zweck verfügt bei einer bevorzugten Ausführungsform das Handinstruments über Hohlräume und Flüssigkeitsanschlüsse, durch die eine geeignete Kühlflüssigkeit, wie z.B. Wasser, geführt werden kann. Die Kühlflüssigkeit kann dann in dem Handinstrument an geeigneter Stelle abgekühlt werden oder aber in das Versorgungsgerät geleitet werden um dann dort abzukühlen. Die Kühlkreisläufe sind üblicherweise geschlossen ausgeführt.

Weitere Vorteile und Merkmale ergeben sich aus der nachfolgenden Beschreibung bevorzugter Ausführungsformen der erfindungsgemäßen Vorrichtung sowie der erfindungsgemäßen Sonde mit Bezug auf die beigefügten Figuren. Einzelne Merkmale der einzelnen Ausführungsformen können dabei im Rahmen der Erfindung miteinander kombiniert werden.

Es zeigen:
- Fig. 1:: eine schematische Darstellung einer bevorzugten Ausführungsform einer Vorrichtung zur Zertrümmerung eines Körpersteins gemäß der vorliegenden Erfindung und
- Fig. 2: eine Detailansicht aus Figur 1.

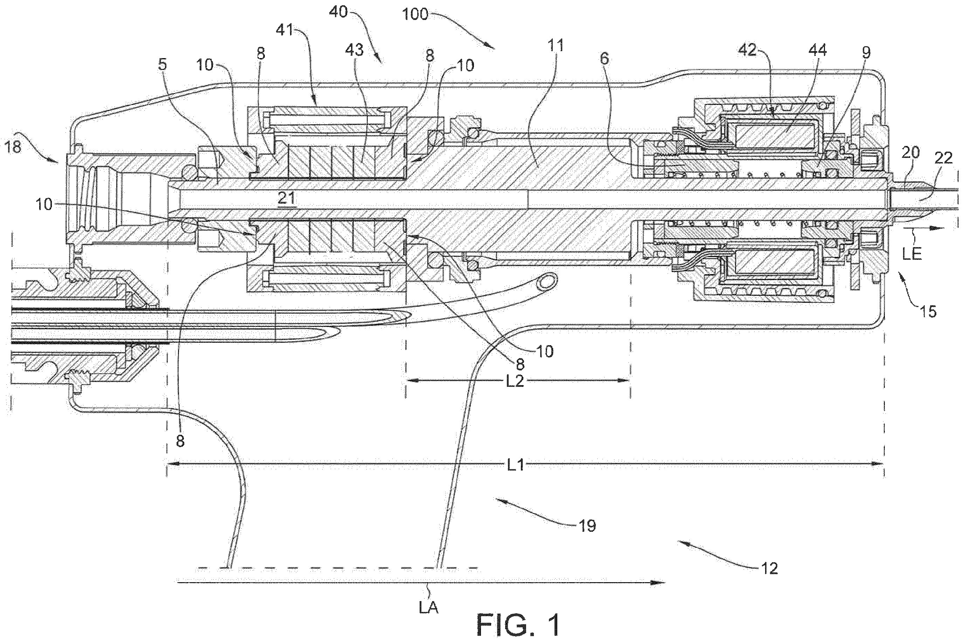

In Figur 1 ist schematisch eine Vorrichtung 100 zur Zertrümmerung und/oder Entfernen von Körpersteinen illustriert. Beispielsweise handelt es sich bei den Körpersteinen um Nieren- oder Harnsteine, die sich mittels der Vorrichtung 100 zertrümmern lassen. Wesentliche Bestandteile einer solchen Vorrichtung 100 sind eine nadelförmige und vorzugsweise hohle Sonde 20 und eine Antriebseinheit 40 zur Auslenkung der Sonde 20 entlang deren Längserstreckung LE. Dabei ist es insbesondere vorgesehen, dass die von Antriebseinheit 40 ausgehenden Anregungen auf die Sonde 20 übertragen werden, wodurch ein im Betrieb dem Körperstein zugewandtes erstes proximales Ende der Sonde 20, das von der Antriebseinheit 40 abgewandt ist, ein Zertrümmern des Körpersteins oder von Teilen des Körpersteins bedingt.

Zum Wechseln zwischen verschiedenen Sondentypen lässt sich die Sonde 20 vorzugsweise über eine Schnittstelle 15 mit der Antriebseinheit 40 lösbar, beispielsweise über ein Gewinde, verbinden. In dem in Figur 1 dargestellten Ausführungsbeispiel ist es vorgesehen, dass die Antriebseinheit 40 in ein Handgerät bzw. Handinstrument 12, insbesondere mit einem Absaugbereich 18, über den insbesondere Körpersteinfragmente absaugbar sind, und einem Griffbereich 19, integriert ist. Weiterhin ist es bevorzugt vorgesehen, dass die Antriebseinheit 40 eine erste Antriebseinrichtung 41 zur periodischen Auslenkung der Sonde 20 und eine zweite Antriebseinrichtung 42 zur impulsförmigen Auslenkung der Sonde 20 umfasst. Besonders bevorzugt ist es vorgesehen, dass die erste Antriebseinrichtung 41 über ein entlang einer Längsachse LA schwingendes Schwingteil 5 auf die Sonde 20 einwirkt und dabei insbesondere eine stehende Welle, insbesondere im Schwingteil 5 und der Sonde 20, ausbildet. Zur Übertragung der von der ersten Antriebseinrichtung 41 ausgehenden Schwingungen, insbesondere Ultraschallschwingungen, sind das Schwingteil 5 und die Sonde 20 in einem betriebsbereiten Zustand in Richtung der Längsachse LA gesehen fluchtend zueinander angeordnet und liegen in einer parallel zur Längsachse LA verlaufenden Richtung gesehen über ihre Stirnseiten aneinander an. Insbesondere sind ein Hohlbereich 21 des Schwingteils 5 und ein weiterer Hohlbereich 22 der Sonde 2 zueinander fluchtend ausgestaltet, um beispielsweise über den Hohlbereich 21 und den weiteren Hohlbereich 22 zertrümmerte bzw. zerkleinerte Fragmente der Körpersteine absaugen bzw. entfernen zu können. Weiterhin ist es vorstellbar, dass die Antriebseinheit 40 zu deren Kühlung eine Kühlvorrichtung (nicht dargestellt) umfasst.

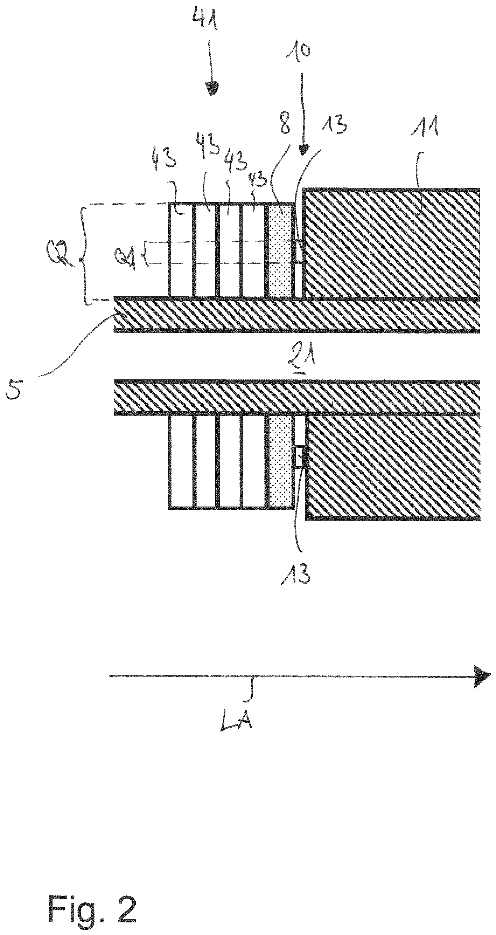

Vorzugsweise weist die erste Antriebseinrichtung 41 einen Piezostack, d. h. eine Anordnung von aneinander anliegenden Piezoelementen 43, auf. Insbesondere sind die gestapelten Piezoelemente 43 als ringförmige Scheiben ausgestaltet, die das Schwingteil 5 in einer senkrecht zur Längsachse LA verlaufenden Ebene umlaufend umgeben. Beispielsweise bilden die gestapelten Piezoelemente 43 einen Teil eines Führungskanals des Schwingteils 5 entlang der Längsachse LA. Dabei durchgreift das Schwingteil 5 die gestapelten Piezoelemente, vorzugsweise mittig. Insbesondere ist es bevorzugt vorgesehen, dass das Schwingteil 5 zur Ausbildung einer stehenden Welle elastisch in der Antriebseinheit 40 gelagert ist. Weiterhin umfasst das Schwingteil 5 einen senkrecht zur Längsachse LA vom einem Grundkörper, insbesondere im Wesentlichen stabförmigen Grundkörper, abstehenden Vorsprung 11 bzw. Mitnehmer. Dieser Vorsprung 11 liegt mit einer in Richtung der Längsachse LA den Vorsprung 11 begrenzenden End- bzw. Stirnseiten an einem Übertragungsbereich 10 an, der wiederum in Richtung der Längsachse LA auf der gegenüberliegende Seite an der Stirnseite der ersten Antriebseinrichtung 41 angeordnet ist (siehe Figur 2). Über den Übertragungsbereich 10 wird eine von der ersten Antriebseinrichtung 41 ausgehende Kraft auf das Schwingteil 5 eingeleitet bzw. weitergeleitet, um im Schwingteil 5 und somit auch in der angrenzenden Sonde 20 eine stehende Welle zu veranlassen.

Neben der ersten Antriebseinrichtung 41 ist weiterhin eine zweite Antriebseinrichtung 42 zur impulsförmigen Auslenkung der Sonde 20 vorgesehen. Die zweite Antriebseinrichtung 42 ist in Richtung der Längsachse LA gesehen versetzt zu der der ersten Antriebseinrichtung 41 angeordnet, wobei die zweite Antriebseinrichtung 42 bevorzugt näher an der Sonde 20 angeordnet ist als die erste Antriebseinrichtung 41. Beispielswiese ist die zweite Antriebseinrichtung 42 an dem der Sonde 20 zugewandten Ende des Schwingteilbereichs 18 und die erste Antriebseinrichtung 41 an dem der Sonde 20 abgewandten Ende des Schwingteilbereichs 42 angeordnet. Insbesondere ist der Vorsprung 11 des Schwingteils 5 in Richtung der Längsachse LA gesehen zwischen der ersten Antriebseinrichtung 41 und der zweiten Antriebseinrichtung 42 angeordnet.

Bevorzugt umfasst die zweite Antriebseinrichtung 42 einen Elektromagneten, der ein Projektil 6 entlang der Längsachse LA beschleunigt. Das Projektil 6 ist vorzugsweise ringscheibenförmig ausgestaltet und ummantelt bzw. umgibt - wie die Piezoelemente 43 - das Schwingteil 5 in einer senkrecht zur Längsachse LA verlaufenden Ebene. Im Betrieb wird das Projektil 6 auf einen Prellkörper 9, beispielsweise einen Amboss, beschleunigt und der Prellkörper 9 überträgt dann den Schlagimpuls beispielsweise auf ein Kragenelement der Sonde 20. Vorzugsweise wird die impulsförmige Auslenkung der Sonde 20 durch die zweite Antriebseinrichtung 42 veranlasst, ohne dass das Schwingteil 5 der ersten Antriebseinrichtung 41 als Kraftflussmittel oder -überträger genutzt wird. Mit anderen Worten: Zur Überlagerung der periodischen und der impulsförmigen Auslenkung wirken die erste Antriebseinrichtung 41 und die zweite Antriebseinrichtung 42 jeweils auf die Sonde 20 ein, ohne sich ein gemeinsames Kraftflussmittel zu teilen.

Es hat sich für eine wirkungsvolle Zertrümmerung von Körpersteinen als besonders vorteilhaft erwiesen, wenn sich die periodische und die impulsförmige Auslenkungen der Sonde 20 gleichzeitig realisieren lassen, d. h. wenn die impulsförmige und die periodische Auslenkungen der Sonde 20 überlagert werden, d. h. impulsförmige und periodische Auslenkung der Sonde 20 zeitgleich realisiert werden. Wegen der vielfach höheren Krafteinwirkung der zweiten Antriebseinrichtung 42 auf die Sonde 20 im Vergleich zu derjenigen der ersten Antriebseinrichtung 41 können impulsförmigen Schwingungen, die durch die zweite Antriebseinrichtung 41 veranlasst werden, die erste Antriebseinrichtung 41 beeinträchtigen. Dies trifft auch zu, wenn die erste Antriebseinrichtung 41 und die zweite Antriebseinrichtung 42 nicht über ein gemeinsames Kraftflussmittel die Auslenkungen der Sonde 20 veranlassen. Hier gelangt die impulsförmige Schwingung beispielsweise über das Gewinde zur der ersten Antriebseinrichtung 41.

Um Schädigung der ersten Antriebseinrichtung 41 entgegenzuwirken, ist es vorgesehen, dass zur Entkopplung von der zweiten Antriebseinrichtung 42 der Übertragungsbereich 1 zur frequenzselektiven Übertragung von Schwingungen, insbesondere als Tiefpassfilter, ausgelegt ist. In der dargestellten Ausführungsform ist der Übertragungsbereich 10 dabei als Querschnittssprung bzw. -verjüngung ausgestaltet. Figur 2 zeigt den Übertragungsbereich 10 zwischen der ersten Antriebseinrichtung 41 und dem Schwingteil 5, insbesondere dem Vorsprung 11 des Schwingteils 5, im Detail. Unter einer Querschnittsverjüngung ist insbesondere zu verstehen, dass sich in der Richtung, entlang der eine Kraft von der ersten Antriebseinrichtung auf das Schwingteil 5 übertragen wird, d. h. entlang der Längsachse, ein Querschnitt, über den die Kraft übertragen wird, ändert, vorzugsweise verkleinert wird. Insbesondere lässt sich die Querschnittsverjüngung quantifizieren durch das Verhältnis des senkrecht zur Längsachse bemessenen ersten Querschnitts Q1 im Übertragungsbereich 10 zu einem senkrecht zur Längsachse LA bemessenen zweiten Querschnitt Q2 der ersten Antriebseinrichtung 41, insbesondere an deren dem Übertragungsbereich 10 zugewandten Stirnseite. Folge einer solchen Querschnittsverjüngung ist eine Reflektion eines möglichst großen Anteils der impulsförmigen Schwingung, die durch die zweite Antriebseinrichtung 42 veranlasst wird und ungewollt andernfalls in die erste Antriebseinrichtung 41 eingespeist werden würde. Dadurch lässt mit Vorteil einer Schädigung der ersten Antriebseinrichtung 41 entgegenwirken.

Beispielswiese lässt sich zur Entkopplung von der zweiten Antriebseinrichtung 42 der Übertragungsbereich 10 an der ersten Antriebseinrichtung 41 stirnseitig (in Richtung der Längsachse gesehen) als Steg ausgestalten. Dabei ist es vorstellbar, dass der Steg in Umlaufrichtung gesehen geschlossen oder unterbrochen ist. Vorstellbar ist es ebenfalls, dass zur Entkopplung von der zweiten Antriebseinrichtung 42 im Übertragungsbereich 10 ein auf eine Resonanzfrequenz der ersten Antriebseinrichtung 41 abgestimmtes Dämpfungselement, beispielswiese in Form einer Unterlegscheibe 13, angeordnet ist. Beispielsweise ist zwischen der ersten Antriebseinrichtung 41 und dem Schwingteil 5 eine Unterlegscheibe 13 angeordnet bzw. verklemmt, über die die von der ersten Antriebseinrichtung 41 ausgehende Bewegungen auf das Schwingteil 5 übertragen werden. Zur Auslegung des Dämpfungselements an die Resonanzfrequenz der ersten Antriebseinrichtung ist es beispielsweise vorgesehen, dass eine Kombination aus elastischen Materialien unterschiedlicher Impedanz zur Ausbildung der Unterlegscheibe 13 vorgesehen ist. Weiterhin ist es bevorzugt vorgesehen, dass - insbesondere ausschließlich - der der Sonde bzw. der zweiten Antriebseinrichtung 42 zugwandte Übertragungsbereich 10 zur frequenzselektiven Übertragung von Schwingungen ausgelegt ist.

Um weiterhin Schädigungen an den Piezoelementen 43 der ersten Antriebseinrichtung zu vermeiden, ist ein Übertragungskörper 8 zwischen den Piezoelement 43 und dem Übertragungsbereich 10 vorgesehen. Der Übertragungskörper 8 ist derart gestaltet, dass er plan und großflächig an dem Piezoelement 43 anliegt. Mit anderen Worten, Der Übertragungsbereich 10 grenzt nicht unmittelbar an das Piezoelement 43, da die im Übertragungsbereich 10 vorgesehene Querschnittsverjüngung andernfalls das Piezoelement 43 zerbrechen lässt.

Weiterhin ist es bevorzugt vorgesehen, dass die von der ersten Antriebseinrichtung 41 ausgehende Bewegung bzw. Kraft in einen Bereich des Schwingteils 5 übertragen wird, indem das Schwingteil 5 angrenzend an den zur frequenzselektiven Übertragung ausgelegte Übertragungsbereich 10 einen senkrecht zur Längsachse LA abstehenden Vorsprung 11 aufweist. Vorzugsweise nimmt ein Verhältnis zwischen einer in Richtung der Längsachse AL bemessene Länge L2 des Vorsprungs 11 zu einer in Richtung der Längsachse LA bemessenen Länge L1 des Schwingteils 5 einen Wert zwischen 0,1 und 0,5, bevorzugt zwischen 0,25 und 0,4 und besonders bevorzugt zwischen 0,28 und 0,38 an.

### Bezugszeichenliste

- 5: Schwingteil
- 6: Projektil
- 8: Übertragungskörper
- 9: Prellkörper
- 10: Übertragungsbereich
- 11: Vorsprung
- 12: Handinstrument
- 13: Unterlegscheibe
- 15: Schnittstellenbereich
- 18: Absaugbereich
- 19: Griffbereich
- 20: Sonde
- 21: Hohlberiech des Schwingteils
- 22: Hohlbereich der Sonde
- 40: Antriebseinheit
- 41: erste Antriebseinrichtung
- 42: zweite Antriebseinrichtung
- 43: Piezoelement
- 44: Elektromagnet
- 100: Vorrichtung
- L1: Länge des Schwingteils
- L2: Länge des Vorsprungs
- LA: Längsachse
- LE: Längserstreckung
- Q1: erster Querschnitt
- Q2: zweiter Querschnitt

## Patentansprüche

1. Vorrichtung (100) zur Zertrümmerung eines Körpersteins, umfassend
- eine Sonde (20) und
- eine Antriebseinheit (40) zur Auslenkung der Sonde (20) entlang deren Längserstreckung (LE),
wobei die Antriebseinheit (40) eine erste Antriebseinrichtung (41) zur periodischen Auslenkung der Sonde (20) und eine zweite Antriebseinrichtung (42) zur impulsförmigen Auslenkung der Sonde (20) umfasst, wobei die Antriebseinheit (40) derart gestaltet ist, dass eine von der zweiten Antriebseinrichtung (41) ausgehende Wirkung auf die erste Antriebseinrichtung (42) reduziert wird, wobei die erste Antriebseinrichtung (41) über ein Schwingteil (5) auf die Sonde (20) einwirkt, **dadurch gekennzeichnet, dass**
die zweite Antriebseinrichtung (42) über einen Prellkörper (9) auf die Sonde (20) einwirkt, wobei die erste Antriebseinrichtung (41) über einen Übertragungsbereich (10) eine die periodische Schwingung veranlassende Kraft auf das Schwingteil (5) überträgt, wobei zur Dämpfung von impulsförmigen Schwingungen der Übertragungsbereich (5) frequenzselektiv, insbesondere als Tiefpassfilter, ausgelegt ist, wobei
der Übertragungsbereich (10) zwischen einem Übertragungskörper (8) der ersten Antriebseinrichtung (41) und dem Schwingteil (5) eine Querschnittsverjüngung, beispielsweise in Form einer Unterlegscheibe (13), aufweist, wodurch die Wirkung, insbesondere Kraftwirkung, von der zweiten Antriebseinheit (42) auf die erste Antriebseinheit (41) reduziert wird.

2. Vorrichtung gemäß Anspruch 1, wobei die Antriebseinheit (40) derart gestaltet ist, dass die periodische Auslenkung und die impulsförmige Auslenkung überlagerbar sind.

3. Vorrichtung (100) gemäß einem der vorhergehenden Ansprüche, wobei der zur frequenzselektiven Übertragung ausgelegte Übertragungsbereich (10) auf einer der zweiten Antriebseinrichtung (42) zugewandten Seite, insbesondere Stirnseite, der ersten Antriebseinrichtung (41) ausgebildet ist.

4. Vorrichtung (100) gemäß einem der vorhergehenden Ansprüche, wobei eine Zusatzmasse zwischen der ersten Antriebseinrichtung (41) und der zweiten Antriebseinrichtung (42) vorgesehen ist, wobei vorzugsweise das Schwingteil (5) einen Grundkörper aufweist, wobei das Schwingteil (5), insbesondere angrenzenden an den zur frequenzselektiven Übertragung ausgelegte Übertragungsbereich (10), einen senkrecht zur Längsachse (LA) abstehenden Vorsprung (11) aufweist.

5. Vorrichtung (100) gemäß einem der vorhergehenden Ansprüche, wobei die erste Antriebseinrichtung (41) ein Piezoelement (43) und/oder die zweite Antriebseinrichtung (42) einen Elektromagneten (44) umfasst.

6. Vorrichtung (100) gemäß einem der vorhergehenden Ansprüche, wobei ein Hohlbereich (22) in der Sonde (20) und ein Hohlbereich (21) im Schwingteil (5) einen Hohlkanal, insbesondere einen Hohlkanal ohne Querschnittsveränderung, zum Absaugen von Körpersteinfragmenten, bilden.

## Claims

1. A device (100) for the fragmentation of a calculus, comprising
- a probe (20) and
- a drive unit (40) for deflecting the probe (20) along its longitudinal extension (LE),
the drive unit (40) comprising a first drive means (41) for the periodic deflection of the probe (20) and a second drive means (42) for the pulsed deflection of the probe (20), the drive unit (40) being configured such that an impact on the first drive means (42) emanating from the second drive means (41) is reduced, the first drive means (41) acting on the probe (20) via an oscillating member (5), **characterized in that**
the second drive means (42) acts on the probe (20) via a baffle (9), the first drive means (41) transmitting a force onto the oscillating member (5) via a transmission region (10), the force causing the periodic oscillation, the transmission region (10) being designed to be frequency-selective, in particular to be a low-pass filter, for attenuating pulsed oscillations, wherein
the transmission region (10) has a cross-sectional tapering, for example in the form of a washer (13), between a transmission body (8) of the first drive means (41) and the oscillating member (5), as a result of which the impact, in particular the force impact, from the second drive means (42) onto the first drive means (41) is reduced.

2. The device according to claim 1, wherein the drive unit (40) is configured such that the periodic deflection and the pulsed deflection are superimposable.

3. The device (100) according to one of the preceding claims, wherein the transmission region (10) designed for frequency-selective transmission is formed on a side, in particular a front side, of the first drive means (41) facing the second drive means (42).

4. The device (100) according to one of the preceding claims, wherein a supplemental mass is provided between the first drive means (41) and the second drive means (42), wherein preferably the oscillating member (5) has a base body, wherein the oscillating member (5) comprises a projection (11) in particular adjacent to the transmission region (10) which is designed for frequency-selective transmission, the projection (11) projecting in a direction perpendicular to the longitudinal axis (LA).

5. The device (100) according to one of the preceding claims, wherein the first drive means (41) comprises a piezo element (43) and/or the second drive means (42) comprises a solenoid (44).

6. The device (100) according to one of the preceding claims, wherein a hollow area (22) in the probe (20) and a hollow area (21) in the oscillating member (5) form a hollow channel, in particular a hollow channel without change in cross-section, for suckingly removing calculus fragments.

## Revendications

1. Dispositif (100) pour la fragmentation d'un calcul corporel, comprenant
- une sonde (20) et
- une unité d'entraînement (40) pour la déviation de la sonde (20) le long de son extension longitudinale (LE),
dans lequel
l'unité d'entraînement (40) comprend un premier organe d'entraînement (41) pour la déviation périodique de la sonde (20) et un deuxième organe d'entraînement (42) pour la déviation impulsionnelle de la sonde (20),
l'unité d'entraînement (40) est conçue de telle sorte qu'un effet, émanant du deuxième organe d'entraînement (42), sur le premier organe d'entraînement (42) soit réduit,
le premier organe d'entraînement (41) agit sur la sonde (20) par l'intermédiaire d'une partie oscillante (5),
**caractérisé en ce que**
le deuxième organe d'entraînement (42) agit sur la sonde (20) par l'intermédiaire d'un corps de rebondissement (9),
le premier organe d'entraînement (41) transmet une force, provoquant l'oscillation périodique, à la partie oscillante (5) par l'intermédiaire d'une zone de transmission (10),
pour amortir des oscillations impulsionnelles, la zone de transmission (5) est conçue de manière sélective en fréquence, en particulier sous forme de filtre passe-bas,
entre un corps de transmission (8) du premier organe d'entraînement (41) et la partie oscillante (5), la zone de transmission (10) présente un rétrécissement en section transversale, par exemple sous la forme d'une rondelle (13), moyennant quoi l'effet, en particulier l'effet de force, du deuxième organe d'entraînement (42) sur le premier organe d'entraînement (41) est réduit.

2. Dispositif selon la revendication 1,
dans lequel l'unité d'entraînement (40) est configurée de manière à ce que la déviation périodique et la déviation impulsionnelle soient superposables.

3. Dispositif (100) selon l'une des revendications précédentes,
dans lequel la zone de transmission (10) conçue pour la transmission sélective en fréquence est réalisée sur une face, en particulier sur une face frontale, du premier organe d'entraînement (41) tournée vers le deuxième organe d'entraînement (42).

4. Dispositif (100) selon l'une des revendications précédentes,
dans lequel
une masse supplémentaire est prévue entre le premier organe d'entraînement (41) et le deuxième organe d'entraînement (42),
de préférence, la partie oscillante (5) présente un corps de base,
la partie oscillante (5), en particulier adjacente à la zone de transmission (10) conçue pour la transmission sélective en fréquence, présente une saillie (11) dépassant perpendiculairement à l'axe longitudinal (LA).

5. Dispositif (100) selon l'une des revendications précédentes,
dans lequel le premier organe d'entraînement (41) comprend un élément piézoélectrique (43), et/ou le deuxième organe d'entraînement (42) comprend un électroaimant (44).

6. Dispositif (100) selon l'une des revendications précédentes,
dans lequel une zone creuse (22) dans la sonde (20) et une zone creuse (21) dans la partie oscillante (5) forment un canal creux, en particulier un canal creux sans modification en section transversale, pour l'aspiration de fragments de calcul corporel.
